(19) Europäisches Patentamt
European Patent Office
Office européen des brevets

(11) Numéro de publication : **0 015 810**
**B1**

(12) # FASCICULE DE BREVET EUROPÉEN

(45) Date de publication du fascicule du brevet : **29.02.84**

(51) Int. Cl.³ : **C 07 H 15/04**, A 61 K 31/70, G 01 N 33/48

(21) Numéro de dépôt : **80400242.6**

(22) Date de dépôt : **20.02.80**

(54) **N-acétyl-L-(N-méthyl-alanyl)-D-isoglutamine et compositions la contenant, pour l'utilisation en tant que régulateurs des mécanismes immunitaires.**

(30) Priorité : **20.02.79 FR 7904316**

(43) Date de publication de la demande : **17.09.80 Bulletin 80/19**

(45) Mention de la délivrance du brevet : **29.02.84 Bulletin 84/09**

(84) Etats contractants désignés : **BE CH DE GB IT NL SE**

(56) Documents cités :
**EP-A- 0 003 833**
**FR-A- 2 349 600**

(73) Titulaire : **ANVAR Agence Nationale de Valorisation de la Recherche**
**13, rue Madeleine Michelis**
**F-92522 Neuilly-sur-Seine (FR)**

(72) Inventeur : **Lefrancier, Pierre**
**20, rue des Bleuets**
**F-91440 Bures sur Yvette (FR)**
Inventeur : **Audibert, Françoise**
**44, rue Ybry**
**F-92200 Neuilly sur Seine (FR)**
Inventeur : **Choay, Jean**
**130, Faubourg Saint-Honoré**
**F-75008 Paris (FR)**
Inventeur : **Chedid, Louis**
**16-18, rue Gaston de Caillavet**
**F-75015 Paris (FR)**
Inventeur : **Lederer, Edgar**
**9, Boulevard Colbert**
**F-92330 Sceaux (FR)**

(74) Mandataire : **Peaucelle, Chantal et al**
**Cabinet Plasseraud 84, rue d'Amsterdam**
**F-75009 Paris (FR)**

EP 0 015 810 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

# N-acétyl-L-(N-méthyl-alanyl)-D-isoglutamine et compositions la contenant, pour l'utilisation en tant que régulateurs des mécanismes immunitaires

L'invention est relative à un composé doué de propriétés biologiques et pharmacologiques de grande valeur, et aux compositions le contenant pour son utilisation en tant que régulateur des mécanismes immunitaires, notamment en tant qu'amplificateur de l'effet immunogène de principes actifs de vaccins administrés à un hôte, animal ou humain.

On sait que des efforts considérables sont consacrés depuis longtemps à la recherche d'agents doués de propriétés régulatrices des réactions immunitaires. Ces recherches ont conduit tout d'abord à la préparation de produits naturels extraits notamment de fragments de peptidoglycanes des parois de mycobactéries, puis à des molécules synthétiques à molécules relativement petites. On dispose ainsi de séries de produits aux propriétés très intéressantes.

Au cours de ces recherches, en même temps que ces nouveaux produits, sont apparues de nouvelles propriétés et, par là même, de nouvelles possibilités d'utilisation. En retour, la nécessité s'est fait sentir de trouver des produits répondant au mieux aux applications découlant de la mise en œuvre de ces propriétés nouvelles, soit qu'il s'agisse de favoriser au maximum un ou plusieurs types d'activité, soit que l'on souhaite scinder les différentes activités possibles pour disposer de produits à action très spécifique, soit encore que l'on veuille réduire ou supprimer certains effets secondaires indésirables.

Pour parvenir à ces objectifs, on s'est efforcé de modifier certaines formes d'administration, mais surtout des recherches ont été entreprises pour modifier l'activité des composés en question en faisant varier certains éléments de leur structure.

Dans les travaux antérieurs, certains auteurs ont tenté d'établir des relations entre certaines configurations ou éléments de structure et l'apparition de propriétés données, ou encore entre diverses propriétés de ce type de produits. C'est ainsi que des chercheurs japonais ont remarqué, dans une publication (SHOZO KOTANI et coll., Biken Journal, Vol. 19, 9-13, 1976), qu'il existait une corrélation apparente entre l'effet adjuvant de l'immunité de ces substances et l'effet pyrogène qui peut apparaître dans certaines conditions expérimentales, notamment pour l'administration de doses élevées.

SHOZO KOTANI et coll. ont émis l'hypothèse que cette pyrogénicité pouvait éventuellement être attribuée à la parenté entre les composés en question et les fragments de peptidoglycanes qui peuvent être obtenus à partir de bactéries gram-positif. Toujours dans cette publication, les auteurs supposent qu'il pourrait exister une relation entre les mécanismes intervenant dans les réponses immunologiques sous l'effet d'une stimulation antigénique et ceux intervenant dans la régulation de la température du corps ou de la réponse fébrile. Ils constatent, en effet, que parmi les composés qu'ils ont testés, les plus adjuvants sont aussi les plus pyrogènes.

Le caractère pyrogène de ces substances peut constituer un inconvénient non négligeable. Si ce caractère est très fortement marqué, l'administration de la substance peut déclencher, chez le sujet traité, un dangereux phénomène de choc thermique. Par ailleurs, même lorsque le caractère pyrogène est faible ou ne se manifeste que pour des doses relativement élevées, il entraîne néanmoins des limitations d'emploi qui peuvent être gênantes. Les doses utilisées doivent être contrôlées de façon rigoureuse, ou encore, certains modes d'administration, en particulier de la voie intraveineuse, doivent être évités.

Pour ces raisons, il est toujours important de disposer de nouvelles substances de ce type offrant un choix élargi de combinaisons de propriétés et permettant ainsi une meilleure adaptation à l'utilisation projetée. C'est dans ce sens qu'ont été entreprises les recherches ayant abouti à l'invention.

Une amélioration visée par les recherches qui ont conduit à la présente invention était l'accroissement de l'activité des composés de ce type pour une dose déterminée, notamment en s'efforçant de réduire la vitesse de catabolisme du produit par l'organisme de l'hôte auquel il est administré. De façon plus particulière, un but de ces recherches était la synthèse de produits résistant mieux à l'hydrolyse enzymatique.

Un autre but de l'invention était de permettre éventuellement la mise en évidence de nouvelles propriétés pharmacologiques ou combinaisons de propriétés, ou encore leur accroissement jusqu'à un niveau non encore obtenu jusqu'à présent. Dans cette optique, il était particulièrement souhaitable de disposer de produits dont l'indice thérapeutique, c'est-à-dire le rapport des doses efficaces aux doses limites pour lesquelles des phénomènes indésirables commencent à apparaître, soit aussi grand que possible.

Les améliorations et modifications souhaitées ont été atteintes, au moins en partie, par les produits faisant l'objet de la présente invention, notamment en ce que l'on obtient des produits adjuvants qui ne manifestent aucun effet pyrogène, ce qui facilite son utilisation en laissant une large latitude de mode d'administration (notamment par voie intraveineuse) et de dose administrée sans risque d'effets secondaires néfastes.

Le produit pour l'utilisation selon l'invention est constitué par la N-acétyl-muramyl-L-(N-méthyl-alanyl)-D-isoglutamine, de formule

(voir figure page 2)

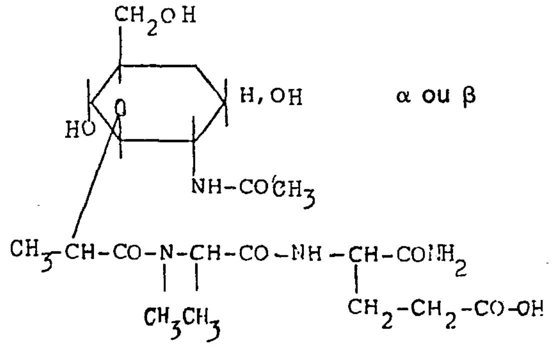

α ou β

(I)

Les compositions selon l'invention peuvent être utilisées en tant que réactifs biologiques, par exemple des adjuvants immunologiques standards, qui peuvent être constitués à l'aide du composé selon l'invention, notamment en vue d'étudier les éventuelles propriétés adjuvantes de substances à l'étude, par comparaison à de tels adjuvants standards ou, au contraire, comme agent susceptible de s'opposer à certains effets liés à l'administration de substances immunosuppressives.

Plus particulièrement, l'invention concerne des médicaments renfermant, à titre de principe actif, le composé sus-défini, ce médicament étant applicable en tant que régulateur de la réponse immunitaire du sujet auquel il est administré.

Ces médicaments sont notamment applicables en tant qu'adjuvant de l'immunité spécifique pour le traitement des maladies infectieuses d'origine bactérienne ou parasitaire ou virale.

L'application de ces médicaments se fait à titre préventif. C'est le cas en particulier lorsque l'on recherche un renforcement de la réponse immunitaire vis-à-vis d'un agent immunogène. L'utilisation d'un agent stimulant le système immunitaire est notamment nécessaire lorsque l'agent immunogène est faible par nature ou provient d'un agent fort dont le caractère immunogène a été amoindri, par exemple au cours de purifications ou modifications antérieures. L'utilisation de l'agent stimulant peut également être utile si l'on souhaite utiliser de très faibles doses d'antigènes forts.

L'invention concerne plus particulièrement les produits ou compositions sus-visés pour leur application à l'amplification de l'effet immunogène de principes actifs de vaccins administrés à un hôte, animal ou humain, notamment dans le cas où ces principes vaccinants appartiennent aux catégories d'agents immunogènes rappelés ci-dessus. En conséquence, l'invention concerne également encore des compositions pharmaceutiques dont le principe actif est constitué par la N-acétyl-L-(N-méthyl-alanyl)-D-isoglutamine, en association avec le véhicule pharmaceutique approprié au mode d'administration requis ou utilisable eu égard à la nature du principe vaccinant utilisé.

Les médicaments selon l'invention peuvent être administrés à l'hôte — animal ou être humain — de toute façon appropriée à l'obtention de l'effet désiré.

L'invention concerne naturellement aussi les diverses compositions pharmaceutiques auxquelles le composé selon l'invention peut être incorporé, le cas échéant en association avec d'autres substances actives. Il est en particulier avantageusement associé à des agents immunogènes, qu'il s'agisse par exemple d'agents immunogènes qui ne peuvent être utilisés qu'à de très faibles doses, notamment en raison de leur mauvaise tolérance, ou d'agents immunogènes faibles.

Des compositions pharmaceutiques avantageuses sont constituées par des solutions ou suspensions injectables contenant une dose efficace du produit selon l'invention. De préférence, ces solutions ou suspensions sont réalisées dans une phase aqueuse stérilisée isotonique, de préférence saline ou glucosée.

L'invention concerne plus particulièrement de telles suspensions ou solutions qui sont aptes à être administrées par injections intradermiques, intramusculaires ou sous-cutanées, ou encore par scarifications.

D'autres compositions pharmaceutiques avantageuses sont constituées par les formes liposomiques des composés selon l'invention. Comme on le sait, les liposomes, en raison de la nature lipidique (et notamment phospholipidiques) des éléments entrant dans leur composition, constituent, pour certains cas, une présentation particulièrement appropriée.

L'invention concerne aussi des compositions pharmaceutiques administrables par d'autres voies, notamment par voie orale ou rectale, ou encore sous des formes destinées à venir en contact avec des muqueuses, notamment les muqueuses oculaires, nasales, pulmonaires ou vaginales.

En conséquence, elle concerne des compositions pharmaceutiques dans lesquelles le composé selon l'invention se trouve associé à des excipients pharmaceutiquement acceptables, solides ou liquides, adaptés à la constitution de forme orale, oculaire ou nasale, ou avec des excipients adaptés à la constitution de formes d'administration rectale, ou encore avec des excipients adaptés à l'administration vaginale, par exemple gélatineux. Elle concerne enfin des compositions destinées à la voie pulmonaire, notamment des solutions préparées en vue de l'administration au moyen d'un appareil d'aérosol classique.

A titre d'exemples de doses du composé susceptibles d'induire une action de renforcement des

défenses immunitaires de l'hôte, lorsqu'administrées sous l'une des formes d'administration évoquée ci-dessus, on mentionnera des doses de 10 à 1 000 µg par kilogramme de corps, par exemple de 50 µg, lorsque l'administration est effectuée par voie parentérale, ou encore d'une dose de 200 à 20 000 µg par kilogramme de corps, par exemple de 1 000 µg, pour d'autres modes d'administration tels que par exemple la voie orale.

L'invention est décrite de façon plus détaillée dans l'exemple qui suit relatif à la préparation de la N-acétyl-muramyl-L-(N-méthyl-alanyl)-D-isoglutamine et des compositions selon l'invention, et à divers essais concernant les propriétés pharmacologiques de ce produit.

I. Préparation de la N-acétyl-muramyl-L-(N-méthyl-alanyl)-D-isoglutamine.

La synthèse est faite de la façon décrite par LEFRANCIER et col. (Int. J. of Peptide and Prot. Res., 9, 1977, 249) en remplaçant la L-alanine par la N-méthyl-L-alanine.

Le pouvoir rotatoire du produit obtenu est $[\alpha]_D^{20} = +19{,}8$ (acide acétique).

L'analyse élémentaire du produit est pour $C_{20}H_{34}N_4O_{11}$ 0,82 $H_2O$ (P.M. 521,28).

calculé C 46,07 H 6,89 N 10,75
trouvé C 46,04 H 6,51 N 10,75

II. Propriétés pharmacologiques.

1. Caractère adjuvant en phase aqueuse et en émulsion

a) En phase aqueuse

Des groupes de 8 souris Swiss âgées de deux mois reçoivent, par injection sous-cutanée (SC), 0,5 mg d'antigène constitué par de l'albumine de sérum bovin (BSA) avec (0,1 mg) ou sans la substance essayée dans une solution saline isotonique. Cette dose élevée d'antigène, parce qu'elle se situe à la limite de la dose paralysante vis-à-vis de la réponse immunitaire, entraîne, de ce fait, une réponse faible ou nulle à l'antigène seul chez les témoins ; elle constitue donc un critère sévère pour mettre en évidence l'activité d'une substance adjuvante. Trente jours plus tard, les souris reçoivent, par la même voie d'administration, un rappel contenant 0,1 mg du même antigène.

Le taux d'anticorps est déterminé, avant et six jours après le rappel, par hémagglutination passive en utilisant des hématies de mouton traitées à la formaline et recouvertes de l'antigène étudié selon la méthode décrite par A. A. HIRATA et M. W. BRANDISS (J. Immunol., *100*, 641-648, 1968).

Le titre en anticorps, représenté par la dilution sérique maximale agglutinant une quantité donnée d'hématies de mouton, atteint au maximum au 36[e] jour suivant la première injection et s'établit de la façon suivante.

| Produits testés | Titre hémagglutinant $Log_2$<br>Réponse primaire J 28 | Titre hémagglutinant $Log_2$<br>Réponse secondaire J 36 |
|---|---|---|
| Témoins | < 1,64 | 3,57 ± 1,78 |
| MDP * | 3,64 | 8,34 ± 1,34 |
| MDP-(N-méthyl-Ala) ** | 4,64 | 9,27 ± 1,19 |

* N-acétyl-muramyl-L-alanyl-D-isoglutamine
** N-acétyl-muramyl-L-(N-méthyl-alanyl)-D-isoglutamine

Dans ce tableau, on constate que le produit en question présente un effet adjuvant sensible qui peut même être supérieur à celui du MDP, notamment en ce qui concerne la réponse primaire. Vis-à-vis du MDP, cependant, l'avantage principal réside dans le caractère extrêmement peu pyrogène de ces produits.

b) En émulsion

Les essais sont effectués sur des lots de 6 cobayes Hartley mâles de 350 g. L'administration est faite par injection intradermique dans le coussinet plantaire de chacune des pattes postérieures. L'ovalbumine (constituant l'antigène) à raison de 1 mg est préparée dans 0,1 ml d'une émulsion de solution isotonique saline, dans une phase huileuse constituée par l'adjuvant incomplet de Freund (FIA). Le composé selon l'invention est administré dans l'émulsion contenant le FIA aux doses indiquées dans le tableau de résultats.

Dix-huit jours après cette immunisation, on recherche d'éventuelles réactions d'hypersensibilité retardée (HSR) à l'antigène en injectant par voie intradermique 0,025 mg d'ovalbumine sur le flanc des animaux, et l'on observe, 48 heures après, la réaction au point d'injection. On mesure le diamètre en millimètres de la réaction ainsi provoquée.

Vingt et un jours après l'injection, les animaux sont saignés. Sur le sérum recueilli, on mesure la teneur en anticorps spécifiques de l'ovalbumine par précipitation du complexe anticorps-antigène dans la zone d'équivalence. La quantité d'azote protéique contenue dans ce précipité est évaluée suivant la méthode de Folin. Les valeurs moyennes des teneurs en anticorps sont indiquées dans le tableau des résultats par le logarithme à base 2. Ces valeurs expriment la quantité, en microgrammes d'azote précipitable par l'antigène, par millilitre de sérum.

Les résultats de ces essais sont les suivants.

| Produits testés | nm ∅ induration après 48 h | Anticorps µg/ml $Log_2$ |
|---|---|---|
| Témoins FIA | 0 | 9,03 ± 0,68 |
| MDP (100 µg) | 11,5 ± 2,5 | 12,31 ± 1,01 |
| MDP-(N-méthyl-Ala) (100 µg) | 14 ± 0,8 | 12,51 ± 0,88 |

Comme précédemment, les résultats obtenus avec les compositions selon l'invention montrent la présence d'un effet adjuvant notable. En outre, comme on le verra plus loin, la comparaison est à l'avantage des compositions selon l'invention lorsque l'on compare les indices thérapeutiques, notamment celui correspondant au rapport activité/effet pyrogène.

2. Etude de la toxicité et de l'effet pyrogène

On a étudié la toxicité des produits par administration parentérale à des souris surrénalectomisées, c'est-à-dire dans des conditions où elles sont particulièrement sensibles aux endotoxines.

On constate que les produits des compositions selon l'invention, aux doses où ils manifestent leurs propriétés, ne sont pas toxiques.

Par ailleurs, on a recherché l'éventualité d'un effet pyrogène chez le lapin en suivant le protocole de la Pharmacopée Européenne, Vol. 2, 1971, pages 58-60. Les essais réalisés notamment avec la N-acétyl-muramyl-N-méthyl-L-alanyl-D-isoglutamine ou l'ester méthylique du même produit montrent l'absence de caractère pyrogène. En particulier, même pour des doses élevées que 10 mg/kg d'animal de N-acétyl-muramyl-N-méthyl-L-alanyl-D-isoglutamine, l'essai reste négatif ; on ne constate pas d'hyperthermie chez le lapin traité.

En conséquence, l'indice thérapeutique dose adjuvante/dose pyrogène des produits selon l'invention est d'un ordre de grandeur très supérieur à celui des produits adjuvants de ce type antérieurement connus. Les produits selon l'invention sont donc particulièrement avantageux.

## Revendications

1. Composé du type muramyl-peptide pour l'utilisation en tant que régulateur des mécanismes immunitaires, caractérisé en ce qu'il consiste en la N-acétyl-muramyl-L-(N-méthyl-alanyl)-D-isoglutamine.

2. Composition pharmaceutique, caractérisée en ce qu'elle contient une dose efficace du composé selon la revendication 1, en association avec des véhicules ou excipients pharmaceutiquement acceptables.

3. Composition pharmaceutique selon la revendication 2, constituée par une solution ou suspension du ou des composés dans un milieu injectable.

4. Composition pharmaceutique selon la revendication 3, caractérisée en ce que le milieu injectable est constitué par une solution aqueuse pour injection, notamment une solution isotonique saline ou glucosée et stérile.

5. Composition pharmaceutique selon la revendication 2, caractérisée en ce que le composé est associé à des excipients respectivement adaptés à l'administration orale, à l'application sur les muqueuses oculaires, des voies respiratoires ou vaginales, ou l'administration rectale.

6. Composition pharmaceutique selon la revendication 2, caractérisée en ce qu'elle est présentée sous forme de liposome.

7. Composition pharmaceutique selon la revendication 2, caractérisée en ce que, pour une administration parentérale, les doses unitaires contiennent de 10 à 1 000 µg de composé.

8. Composition pharmaceutique selon la revendication 2, caractérisée en ce que, pour une administration orale, les doses unitaires contiennent de 200 à 20 000 µg de composé.

9. Composition pharmaceutique selon l'une quelconque des revendications 2 à 8, caractérisée en ce

qu'elle contient en outre un principe actif de vaccin.

10. Réactif de laboratoire comprenant un composé selon la revendication 1.

## Claims

1. Compound of the muramyl-peptide type for use as a regulator of immunitary mechanisms, characterized in that it consists of N-acetyl-muramyl-L-(N-methyl-alanyl)-D-isoglutamine.

2. Pharmaceutical composition characterized in that it contains an efficient dosage of the compound according to claim 1, associated with pharmaceutically acceptable vehicles or carriers.

3. Pharmaceutical composition according to claim 2, constituted by a solution or suspension of the compound or compounds in an injectable medium.

4. Pharmaceutical composition according to claim 3, characterized in that the injectable medium is constituted by an aqueous solution for injection, particularly a saline or glucosed isotonic and sterile solution.

5. Pharmaceutical composition according to claim 2, characterized in that the compound is associated with carriers respectively suitable for oral administration, application on ocular mucosae, respiratory or vaginal tracts, or for rectal administration.

6. Pharmaceutical composition according to claim 2, characterized in that it is presented in the form of liposomes.

7. Pharmaceutical composition according to claim 2, characterized in that, for parenteral administration, the unit dosages contain from 10 to 1,000 μg of compound.

8. Pharmaceutical composition according to claim 2, characterized in that, for oral administration, the unit dosages contain from 200 to 20,000 μg of compound.

9. Pharmaceutical composition according to anyone of claims 2 to 8, characterized in that it further contains an active principle of a vaccine.

10. Laboratory reagent comprising a compound according to claim 1.

## Ansprüche

1. Verbindung vom Muramyl-Peptid-Typ zur Verwendung als Regulator der Immunmechanismen, dadurch gekennzeichnet, daß sie aus N-Acetyl-muramyl-L-(N-methyl-alanyl)-D-isoglutamin besteht.

2. Pharmazeutische Zusammensetzung, dadurch gekennzeichnet, daß sie eine wirksame Dosis der Verbindung gemäß Anspruch 1 enthält, zusammen mit einem Träger oder pharmazeutisch annehmbaren Exzipienten.

3. Pharmazeutische Zusammensetzung nach Anspruch 2, dadurch gekennzeichnet, daß sie aus einer Lösung oder Suspension der Verbindungen in einem injizierbaren Medium besteht.

4. Pharmazeutische Zusammensetzung nach Anspruch 3, dadurch gekennzeichnet, daß das injizierbare Medium aus einer wäßrigen Lösung zur Injektion besteht, insbesondere aus einer sterilen isotonischen Salz- oder Glukoselösung.

5. Pharmazeutische Zusammensetzung nach Anspruch 2, dadurch gekennzeichnet, daß sie die Verbindung zusammen mit Exzipienten enthält, die für eine orale Administration, eine Applikation in die Augenschleimbeutel oder Luftwege, oder für eine vaginale oder rektale Verabreichung geeignet sind.

6. Pharmazeutische Zusammensetzung nach Anspruch 2, dadurch gekennzeichnet, daß sie in Form von Liposomen vorliegt.

7. Pharmazeutische Zusammensetzung nach Anspruch 2, dadurch gekennzeichnet, daß für die parenterale Administration die Einzeldosen 10 bis 1 000 μg der Verbindung enthalten.

8. Pharmazeutische Zusammensetzung nach Anspruch 2, dadurch gekennzeichnet, daß für eine orale Administration die Einzeldosen 200 bis 20 000 μg der Verbindung enthalten.

9. Pharmazeutische Zusammensetzung nach einem der Ansprüche 2 bis 8, dadurch gekennzeichnet, daß sie außerdem einen aktiven Impfstoffbestandteil enthält.

10. Laboratoriumsreagenz enthaltend eine Verbindung nach Anspruch 1.